(19) Europäisches Patentamt

European Patent Office

Office européen des brevets

(11) EP 1 704 853 A2

(12) EUROPEAN PATENT APPLICATION

(43) Date of publication:
27.09.2006 Bulletin 2006/39

(51) Int Cl.:
*A61K 8/67* (2006.01)

(21) Application number: 06251578.8

(22) Date of filing: 23.03.2006

(84) Designated Contracting States:
AT BE BG CH CY CZ DE DK EE ES FI FR GB GR
HU IE IS IT LI LT LU LV MC NL PL PT RO SE SI
SK TR
Designated Extension States:
AL BA HR MK YU

(30) Priority: 24.03.2005 US 89259

(71) Applicant: NEUTROGENA CORPORATION
Los Angeles
California 90045 (US)

(72) Inventors:
• Singleton, Laura
  Los Angeles, CA 90043 (US)
• Barkovic, Sylvia
  Long Beach, CA 90814 (US)
• Fernandez, Aldo O.
  Los Angeles, CA 90043 (US)
• Martens, Nicolas
  Hermosa Beach, CA 90254 (US)

(74) Representative: Fisher, Adrian John et al
Carpmaels & Ransford,
43-45 Bloomsbury Square
London WC1A 2RA (GB)

(54) **Water-in-silicone emulsion compositions**

(57) The present invention relates to a composition containing a retinoid and/or peptide complexed with a copper ion wherein the composition is a water-in-silicone emulsion, and the use thereof.

EP 1 704 853 A2

**Description**

## BACKGROUND OF THE INVENTION

An emulsion is generally a two-phase system that is prepared by combining two immiscible liquids, one of which is dispersed throughout the other (e.g., in the form of globules). The mixture is generally prevented from separating into distinct phases by the addition of an emulsifying agent or emulsifier. Emulsions are considered desirable as topical preparations due to their aesthetically pleasing appearance.

Film-formers are materials that produce a continuous film on skin, hair, or nails. When film-formers are added to topical preparations and applied to skin, a tightening effect on the skin can be observed. Also, film-formers are considered desirable due to their ability to maintain an active ingredient's contact with the skin.

The present invention relates to the discovery that water-in-silicone emulsions are unexpectedly effective for the topical administering retinoids as well as copper containing peptides. Such emulsions were unexpectedly found to enhance the permeation of retinoids into the skin and stabilize copper-containing peptides. The optional presence of a film-former can also provide for reduction in irritation as well as both short and long term benefits of the composition, such as skin tightening, cheek and under eye firmness, improved cheek and jowl contour, and reduction of the appearance of fine lines and wrinkles.

## SUMMARY OF THE INVENTION

The present invention relates to a composition containing (i) a retinoid and/or (ii) a peptide complexed with a copper ion wherein the composition is a water-in-silicone emulsion, and the use thereof.

Other features and advantages of the present invention will be apparent from the detailed description of the invention and from the claims.

## DETAILED DESCRIPTION OF THE INVENTION

It is believed that one skilled in the art can, based upon the description herein, utilize the present invention to its fullest extent. The following specific embodiments can be construed as merely illustrative, and not limitative of the remainder of the disclosure in any way whatsoever.

Unless defined otherwise, all technical and scientific terms used herein have the same meaning as commonly understood by one of ordinary skill in the art to which the invention belongs. Also, all publications, patent applications, patents, and other references mentioned herein are incorporated by reference. As used herein, all percentages are by weight unless otherwise specified.

Definitions

As used herein, "topical application" and "topically applying" means directly laying on or spreading on the skin, hair, or nail, e.g., by use of the hands or an applicator such as a wipe.

As used herein, "cosmetically-acceptable" means that the retinoid, copper peptide, cosmetically active agents, inert ingredients, or composition which the term describes are suitable for use in contact with tissues (e.g., the skin) without undue toxicity, incompatibility, instability, irritation, allergic response, and the like, commensurate with a reasonable benefit/risk ratio.

As used herein, "safe and effective amount" means an amount of compound (e.g., the retinoid or copper peptide) or composition sufficient to significantly induce a positive modification in the condition to be regulated or treated, but low enough to avoid serious side effects. The safe and effective amount of the compound or composition will vary with the particular condition being treated, the age and physical condition of the end user, the severity of the condition being treated/prevented, the duration of the treatment, the nature of concurrent therapy, the specific compound or composition employed, the particular cosmetically-acceptable topical carrier utilized, and like factors.

As used herein, "substantially free" means less than about 1%, by weight, preferably less than about 0.5%, and most preferably none.

Unless otherwise stated, where the compounds recited herein have at least one chiral center, they may accordingly exist as enantiomers. Where the compounds possess two or more chiral centers, they may additionally exist as diastereomers. It is to be understood that all such isomers and mixtures thereof are encompassed within the scope of the present invention. For example, retinoic acid includes, but is not limited to, all-trans retinoic acid and isotretinoin. Furthermore, some of the crystalline forms for the compounds may exist as polymorphs and as such are intended to be included in the present invention. In addition, some of the compounds may form solvates with water (i.e., hydrates) or common organic solvents, and such solvates are also intended to be encompassed within the scope of this invention.

Water-in-silicone Emulsion

Water-in-silicone emulsions according to the present invention includes two liquid phases, (i) a phase containing a liquid silicone and other lipophilic materials ("silicone phase" or "oil phase") and (ii) a phase containing water and other hydrophilic materials ("water phase" or "aqueous phase"). The silicone phase generally is from about 10 to about 70 percent by weight of the emulsion, and the water phase generally is from about 30 to about 90 percent by weight of the emulsion, based on the total weight of the emulsion.

The silicone phase includes a liquid silicone. Examples of liquid silicones include, but are not limited to, dimethicones and cyclomethicones (such as cyclopentasiloxanes and cyclohexasiloxanes), phenyltrimethicones, caprylylmethicones, trisiloxane (and) dimethicone, cyclomethicone (and) dimethiconol, dimethicone (and) dimethiconol.

In addition to the liquid silicone, the silicone phase may also contain one or more other non-silicone oils (such as mineral oil), thickeners, water-in-silicone emulsifiers, oil-soluble film-formers, oil-soluble antioxidants, humectants, oil-soluble anti-irritants (such as bisabolol), and silicone waxes (such as Cetyl Dimethicone).

Examples of thickeners include, but are not limited to, silicone thickeners such as dimethicone crosspolymers such as dimethicone/vinyl dimethicone crosspolymer and dimethicone/phenyl vinyl dimethicone crosspolymer. The thickeners typically will be present in the composition in an amount from about 0.001% to about 30% by weight, in particular in an amount from about 0.01% to about 12% by weight.

Examples of water-in-silicone emulsifiers include, but are not limited to, dimethicone PEG 10/15 crosspolymer, dimethicone copolyol, cetyl dimethicone copolyol, and PEG-15 lauryl dimethicone crosspolymer, laurylmethicone crosspolymer, cyclomethicone and dimethicone copolyol, dimethicone copolyol (and) caprylic/capric triglycerides, polyglyceryl-4 isostearate (and) cetyl dimethicone copolyol (and) hexyl laurate, and dimethicone copolyol (and) cyclopentasiloxane. The water-in-silicone emulsifiers typically will be present in the composition in an amount from about 0.001% to about 10% by weight, in particular in an amount from about 0.01% to about 5.75% by weight.

Examples of oil-soluble film-formers include, but are not limited to, adipic acid/diethylene glycol/glycerin crosspolymer, acrylates/dimethicone copolymer, trimethyl siloxysilicate, VP/Hexadecene copolymer, and TVP/Eicocene. The oil-soluble film-formers typically will be present in the composition in an amount from about 0.001% to about 5% by weight, in particular in an amount from about 0.01% to about 2% by weight.

The water phase includes water. In addition to water, the water phase may also contain one or more water-soluble film-formers. Examples of water-soluble film-formers include, but are not limited to, gums such as acacia senegal gum and xanthan gums, high molecular weigh proteins such as hydrolyzed soy skin proteins, and polymers such as chitosan polymers (e.g., chitosan lactate and glycolate). The water-soluble film-formers typically will be present in the composition in an amount from about 0.001% to about 10% by weight, in particular in an amount from about 0.01% to about 1% by weight.

Retinoids

In one embodiment, the compositions of the present invention contain one or more retinoids. Examples of retinoids include, but are not limited to, retinol, retinal, retinoic acid, etretinate, acitretin, adapalene, tazarotene, and alitretinoin, and salts and esters thereof, such a retinyl palmitate and retinyl acetate.

In one embodiment, the composition comprises a safe and effective amount of the retinoid. The retinoid typically will be present in the composition in an amount from about 0.001% to about 10% by weight, in particular in an amount from about 0.01% to about 1% by weight.

Peptide Complexed with a Copper Ion

In one embodiment, the compositions of the present invention contain one or more cooper peptides. What is meant by a "copper peptide" is a peptide complexed with a copper ion. Examples of such copper peptides are set forth in U.S. Patent Nos. 4,665,054, 4,760,051, 4,810,693, 4,877,770, 5,135,913, 5,348,943, 5,382,431, and 5,550,183. In one embodiment, the peptide has from 3 to 10 amino acids. In one embodiment, the peptide is of the formula 1:

$$[ \begin{smallmatrix} R1 \\ > \\ R2 \end{smallmatrix} A1\text{-}A2\text{-}His\text{-}A3\text{-}A4\text{-}R3]_n : copper\ (II)$$

Formula 1

wherein A1 is Gly or absent; A2 is Gly, Lys, Ala, Ser, or Val; A3 is Lys or Gly; A4 is Trp, $(Gly)_n$-Trp where n is from 1 to 4, Pro-Val-Phe-Val, Val-Phe-Val, or absent; each R1 and R2, independently, is H, $C_{1-12}$ alkyl, $C_{7-10}$ phenylalkyl, or C (=O) $E_1$, where $E_1$ is $C_{1-20}$ alkyl, $C_{3-20}$ alkenyl, $C_{3-20}$ alkynyl, phenyl, 3,4-dihydroxyphenylalkyl, naphthyl, or $C_{7-10}$ phenylalkyl; provided that when either R1 or R2 is C (=O) $E_1$, the other must be H; R3 is OH, $NH_2$, $C_{1-12}$ alkoxy, $C_{7-10}$ phenylalkoxy, $C_{11-20}$ naphthylalkoxy, $C_{1-12}$ alkylamino, $C_{7-10}$ phenylalkylamino, or $C_{11-20}$ naphthylalkylamino; and n is 1 or 2. Copper (II) may be bound to one or more counter anions. Examples of additional counter anions include, but are not limited to, halides such as chloride, acetates, phosphonates, and sulfates, e.g., copper diacetate.

In one embodiment, A1 is absent. In one embodiment, A2 is Gly, Lys, or Ala. In one embodiment, A3 is Lys or Gly. In one embodiment, A4 is absent. In one embodiment, R1 and R2 are both H. In one embodiment, R3 is OH, $NH_2$, or $C_{1-12}$ alkoxy.

In one embodiment, the peptide is $[H_2$-Gly-His-Lys-OH$]_n$: copper (II), $[H_2$-Gly-His-Lys-NH$_2]_n$: copper (II) (Copper Tripeptide-1), $[H_2$-Ala-His-Lys-OH$]_n$:copper (II), or $[H_2$-Ala-His-Lys-NH$_2]_n$: copper (II).

The symbol A1, A2, or the like used herein (e.g., in Formula 1) stands for the residue of an alpha-amino acid. Such symbols represent the general structure, -NH-CH(X)-CO-or =N-CH(X)-CO- when it is at the N-terminus or -NH-CH(X)-CO- when it is not at the N- terminus, where X denotes the side chain (or identifying group) of the alpha-amino acid, e.g., X is $-CH(CH_3)_2$for Val. Note that the N-terminus is at the left and the C-terminus at the right in accordance with the conventional representation of a polypeptide chain. R1 and R2 are both bound to the free nitrogen atom N-terminal amino acid (e.g., A1 or A2) and the $R_3$ is bound to the free carboxy group of the C-terminal amino acid (e.g., A3 or A4). Further, where the amino acid residue is optically active, it is the L-form configuration that is intended unless the D-form is expressly designated. An alkyl group, if not specified, contains 1-12 carbon atoms.

The amount of the copper peptide present in the composition will depend on the copper peptide used and the intended use of the composition. In one embodiment, the composition comprises a safe and effective amount of the copper peptide. The copper peptide typically will be present in the composition in an amount from about 0.001% to about 20% by weight, in particular in an amount from about 0.01% to about 1% by weight.

The method for synthesizing peptides of the present invention are well documented and are within the ability of a person of ordinary skill in the art. The synthesis of copper peptides of the present invention are set forth in U.S. Patent Nos. 4,810,693 and 5,550,183.

Basic Amino Acid

In one embodiment, a composition of the present invention includes both a copper peptide and a basic amino acid. What is meant by a "basic amino acid" is an amino acid that has a second basic group which may be an amino group such as lysine, a guanidino group such as arginine, or an imidazole ring such as histidine. Examples of basic amino acids include the D- and L-isomers of arginine, histidine, and lysine.

The amount of the basic amino acid present in the composition will depend on the basic amino acid used and/or the amount of copper peptide in the composition. In one embodiment, the composition comprises a safe and effective amount of the basic amino acid. The basic amino acid typically will be present in the composition in an amount from about 0.001% to about 20% by weight, in particular in an amount from about 0.01% to about 5% by weight.

Additional Cosmetically Active Agents

In one embodiment, the topical composition further comprises another cosmetically active agent in addition to the copper peptide. What is meant by a "cosmetically active agent" is a compound that has a cosmetic or therapeutic effect on the skin, hair, or nails, e.g., lightening agents, darkening agents such as self-tanning agents, anti-acne agents, shine control agents, antimicrobial agents, anti-inflammatory agents, anti-mycotic agents, anti-parasite agents, external analgesics, sunscreens, photoprotectors, antioxidants, keratolytic agents, detergents/surfactants, moisturizers, nutrients, vitamins, energy enhancers, anti-perspiration agents, astringents, deodorants, hair removers, firming agents, anti-callous agents, and agents for hair, nail, and/or skin conditioning.

In one embodiment, the cosmetically active agent is selected from, but not limited to, the group consisting of hydroxy acids, benzoyl peroxide, sulfur resorcinol, ascorbic acid, D-panthenol, hydroquinone, octyl methoxycinnimate, titanium dioxide, octyl salicylate, homosalate, avobenzone, polyphenolics, carotenoids, free radical scavengers, ceramides, polyunsaturated fatty acids, essential fatty acids, enzymes, enzyme inhibitors, minerals, hormones such as estrogens, steroids such as hydrocortisone, 2-dimethylaminoethanol, copper salts such as copper chloride, coenzyme Q10, lipoic acid, amino acids such a proline and tyrosine, vitamins, lactobionic acid, acetyl-coenzyme A, niacin, riboflavin, thiamin, ribose, electron transporters such as NADH and FADH2, and other botanical extracts such as aloe vera, feverfew, and soy, and derivatives and mixtures thereof. The cosmetically active agent will typically be present in the composition of the invention in an amount of from about 0.001% to about 20% by weight of the composition, e.g., about 0.01% to about 10% such as about 0.1% to about 5%.

Examples of vitamins include, but are not limited to, vitamin A, vitamin Bs (such as vitamin B3, vitamin B5, and vitamin B12), vitamin C, vitamin K, and vitamin E, and derivatives thereof.

Examples of hydroxy acids include, but are not limited, to glycolic acid, lactic acid, malic acid, salicylic acid, citric acid, and tartaric acid.

In one embodiment, the composition contains an antioxidant. Examples of antioxidants include, but are not limited to, water-soluble antioxidants such as sulfhydryl compounds and their derivatives (e.g., sodium metabisulfite and N-acetyl-cysteine), lipoic acid and dihydrolipoic acid, resveratrol, lactoferrin, ascorbic acid, and ascorbic acid derivatives (e.g., ascorbyl palmitate and ascorbyl polypeptide). Oil-soluble antioxidants suitable for use in the compositions of this invention include, but are not limited to, butylated hydroxytoluene, tocopherols (e.g., tocopheryl acetate), tocotrienols, and ubiquinone. Natural extracts containing antioxidants suitable for use in the compositions of this invention, include, but not limited to, extracts containing flavonoids and isoflavonoids and their derivatives (e.g., genistein and diadzein), extracts containing resveratrol and the like. Examples of such natural extracts include grape seed, green tea, pine bark, and propolis. Other examples of antioxidants may be found on pages 1612-13 of the ICI Handbook.

Other Materials

Various other cosmetically-active agents may also be present in the skin care products. These include, but are not limited to, skin protectants, humectants, and emollients. The skin care products may also comprise chelating agents (e.g., EDTA), preservatives (e.g., parabens), pigments, dyes, opacifiers (e.g., titanium dioxide), and fragrances.

Products

The water-in-silicone compositions of the present invention can be used for topical application to skin, hair, and nails. The compositions may be made into a wide variety of product types that include but are not limited to serums, lotions, creams, and make-up (such as foundations, mascaras, and lipsticks).

In one embodiment, the composition and/or product is topically applied to the skin (e.g., the skin of a human in need of such treatment) for use in reducing the appearance of wrinkles (such as crow's feet, fine lines, and deep wrinkles), reducing the appearance of dark under-eye circles, treating acne, treating photodamage, reducing the appearance of hyperpigmentation such as age spots, reducing the appearance of roughness, sallowness, or pores on the skin. The composition may be applied by or be incorporated into, a wipe (such as a non-woven substrate) or a sponge pad.

The composition and products containing such compositions of the present invention may be prepared using methodology that is well known by an artisan of ordinary skill.

Example 1

The following are two examples of a water-in-silicone emulsion of the present invention containing retinol. The ingredients and their corresponding weight percentages are set forth in Table 1.

In the primary container, the following oil phase ingredients were combined: Cyclopentasiloxane (and) Cyclohexasiloxane, Cyclopentasiloxane (and) Dimethicone/Vinyl Dimethicone Crosspolymer, Dimethicone (and) Dimethicone PEG 10/15 Crosspolymer, Adipic Acid/Diethylene Glycol/Glycerin Crosspolymer, Isononyl Isonoanoate, Squalane, Tocopherol, Ethylhexylglycerin, Bisabolol, and BHT. These ingredients were mix until homogeneous.

The following water phase ingredients were combined in a second container: Water, Erythorbic Acid, Dipotassium Glyccyrrhizate, Sodium Citrate, and Sodium Chloride. These ingredients were mixed until completely dissolved. Then the Dipropylene Glycol, Oat (Avena Sativa) Kernel Extract, and Glycerin were added to the second container and mixed until homogeneous.

While mixing the ingredients of the primary container, the mixture in the second container was added very slowly, causing the resulting mixture in the primary container to gel. The resulting mixture was further mixed until the emulsion was well formed. After the emulsion was formed, retinol was added with continuous mixing until homogeneous.

Table 1

| CTFA NAME | Sample A (% Weight) | Sample B (% Weight) |
|---|---|---|
| Water | QS 100 | QS 100 |
| Dipropylene Glycol | 13 | 13 |
| Cyclopentasiloxane (and) cyclohexasiloxane | 17.3 | 17.3 |

(continued)

| CTFA NAME | Sample A (% Weight) | Sample B (% Weight) |
|---|---|---|
| Cyclopentasiloxane (and) dimethicone/vinyl dimethicone crosspolymer | 12 | 12 |
| Dimethicone (and) dimethicone PEG 10/15 crosspolymer | 6.25 | 6.25 |
| Isononyl isononoate | 3 | 3 |
| Adipic acid/diethylene glycol/glycerin crosspolymer | 2 | 2 |
| Squalene | 1 | 1 |
| Bisabolol | 1 | 1 |
| Oat (avena sative) kernel extract | 0.9 | 0.9 |
| Ethylhexylglycerin | 0.7 | 0.7 |
| Glycerin | 0.5 | 0.5 |
| Sodium chloride | 0.5 | 0.5 |
| Sodium citrate | 0.2 | 0.2 |
| Erythorbic Acid | 0.1 | 0.1 |
| Dipottassium Glyccyrrhizate | 0.1 | 0.1 |
| Retinol | 0.075 | 0.1 |
| BHT | 0.7 | 0.7 |
| Tocopherol | 0.5 | 0.5 |

Example 2

The following is an example of a water-in-silicone emulsion of the present invention. The ingredients and their corresponding weight percentages are set forth in Table 2.

In the primary container the silicone phase ingredients, namely Cyclopentasiloxane (and) Cyclohexasiloxane, Cyclopentasiloxane (and) Dimethicone/Vinyl Dimethicone Crosspolymer, Dimethicone (and) Dimethicone PEG 10/15 Crosspolymer, Cyclopentasiloxane and Acrylates/Dimethicone Copolymer, Squalane, Tocopheryl Acetate, Ethylhexylglycerin, Bisabolol, Fragrance, and BHT were combined and mixed until homogeneous.

Water, Copper Tripeptide-1, and L-arginine were separately mixed until the Copper Tripeptide-1 and the arginine were dissolved. The combination of water, acacia Senegal gum, hydrolyzed soy skin protein, and xanthan gum (Skin Tightening Serum, Tri-K Industry, Northvale, NJ) was then added to the Copper Tripeptide-1 mixture, and the resulting mixture was then added to dipropylene glycol and glycerin and mixed until homogeneous, forming such water phase.

While mixing the oil phase, the water phase was slowly added into the oil phase, following which the oil phase had a gelatinous consistency. The resulting water-in-silicone emulsion was mixed until well formed.

Table 2

| CTFA Name | % Weight |
|---|---|
| Water | QS 100 |
| Dipropylene Glycol | 18 |
| Cyclopentasiloxane (and) Dimethicone/Vinyl Dimethicone Crosspolymer | 12 |
| Cyclopentasiloxane (and) Cyclohexasiloxane | 17 |
| Dimethicone (and) Dimethicone PEG 10/15 Crosspolymer/KSG-210 | 5 |
| Squalane | 1 |
| Water, Acacia Senegal Gum, Hydrolyzed Soy Skin Protein, Xanthan Gum | 1 |
| Tocopheryl Acetate | 0.5 |

(continued)

| CTFA Name | % Weight |
|---|---|
| Ethylhexylglycerin | 0.5 |
| Bisabolol | 0.5 |
| Glycerin | 0.5 |
| Copper Tripeptide-1 | 0.35 |
| Fragrance | 0.25 |
| Cyclopentasiloxane (and) Acrylates/Dimethicone Copolymer/KP-545 | 0.25 |
| BHT | 0.07 |
| L-Arginine | 0.03 |

Example 3

The permeation of retinol from the Sample B formulation of Example 1, with and without the film former Adipic Acid/Diethylene Glycol/Glycerin Crosspolymer, was compared in an in-vitro model study with a commercially available oil-in-water emulsion containing 0.1% retinol to study the percutaneous penetration and absorption of retinol for a 24 hour period. In the test, pig skin was washed and the thickness of the skin was measured prior to mounting between the halves of vertical Franz-type diffusion cells (with the stratum corneum facing the donor chamber). The area of diffusion was about 2.54 cm$^2$. The diffusion cells were immersed in a bath maintained at 32°C and the chambers were filled with a PBS solution containing polyethylene glycol 20 oleyl ether and BHT. The products were then weighed and applied to the surface of the skin samples. After 24 hours (in the dark), the formulation remaining on the skin surface and the retinol that has penetrated into the skin was analyzed and quantified by HPLC.

As shown in Table 2 below, the efficiency of retinol permeation into the skin was unexpectedly increased by the water-in-silicone emulsion formulations of Example 1 as compared to the commercially available oil-in-water emulsion.

| TEST PRODUCT | Retinol Permeation |
|---|---|
| Oil-in-Water Formulation | 4.0% |
| Example 1 | 12.4% |
| Example 1 (without film-former) | 18.1% |

Example 4

The irritation potential of the Sample B formulation of Example 1, with and without the film former Adipic Acid/ Diethylene Glycol/Glycerin Crosspolymer, was also tested in a human repetitive patch test model that is used to predict the tolerability of products under normal use. The technique involves application of undiluted test products under semi-occlusive patches daily over a twelve-day test period. Approximately 100 µl of test product is applied to each non-woven cotton pad and secured to the skin with porous, hypoallergenic tape. An undosed patch served as a negative control, while a 1.0% sodium lauryl sulfate (SLS) patch served as the positive control. Each day, patches were removed and test sites professionally graded for irritation prior to re-patching. Grades were compiled over the test period to generate a cumulative irritation score, as depicted below in Table 3. An unexpected, substantial reduction in the irritation was observed when the film former was present in the formulation.

Table 3

| Test Product | Cumulative Irritation Score |
|---|---|
| Example 1: Formula #1156-177 (0.1% retinol serum without film-former) | 204 |
| Example 1: Formula #1204-120 (0.1% retinol serum with film-former) | 108 |
| Undosed - negative control | 0 |

(continued)

| Test Product | Cumulative Irritation Score |
|---|---|
| SLS - positive control* | 514 |
| *Average of two studies | |

Example 5

The stability of the water-in-silicone emulsion of Example 2 was compared to that of the oil-in-water emulsion composition containing 0.3% of Copper Tripeptide-1 ("Oil-in-water Product"). The appearance of the two products, stored in accelerated stability conditions at 40°C, were then compared. After six months of storage, the oil-in-water emulsion turned from a light-blue color to light-green in color, indicating degradation of the Copper-tripeptide 1. On the other hand, Example 1 did not turn green over the same time period, indicating enhanced stability for the water-in-silicone emulsion composition.

It is understood that while the invention has been described in conjunction with the detailed description thereof, that the foregoing description is intended to illustrate and not limit the scope of the invention, which is defined by the scope of the appended claims. Other aspects, advantages, and modifications are within the claims.

**Claims**

1. A composition comprising a retinoid when said composition is a water-in-silicone emulsion.

2. A composition of claim 1, wherein said retinoid is retinol.

3. A composition of claim 2, wherein said composition further comprises a water-soluble film-former.

4. A composition of claim 3, wherein said water-soluble film-former is selected from the group consisting of gums, proteins, and polymers.

5. A composition of claim 2, wherein said composition further comprises an oil-soluble film-former.

6. A composition of claim 3, wherein said composition further comprises an oil-soluble film-former.

7. A composition of claim 5, wherein said oil-soluble film-former selected from the group consisting of adipic acid/diethylene glycol/glycerin crosspolymer, acrylates/dimethicone copolymer, trimethyl siloxysilicate, VP/hexadecene copolymer, and TVP/eicocene copolymer.

8. A composition of claim 6, wherein said oil-soluble film-former selected from the group consisting of adipic acid/diethylene glycol/glycerin crosspolymer, acrylates/dimethicone copolymer, trimethyl siloxysilicate, VP/hexadecene copolymer, and TVP/eicocene copolymer.

9. A composition of claim 2, wherein the external phase of said emulsion comprises a dimethicone crosspolymer.

10. A composition of claim 2, wherein the external phase of said emulsion comprises a dimethicone crosspolymer.

11. A composition comprising a peptide complexed with a copper ion when said composition is a water-in-silicone emulsion.

12. A composition of claim 11, wherein said peptide is of the Formula 1:

```
      R1

  [    >A1-A2-His-A3-A4-R3]_n : copper (II)

      R2
```

Formula 1

wherein A1 is Gly or absent; A2 is Gly, Lys, Ala, Ser, or Val; A3 is Lys or Gly; A4 is Trp, $(Gly)_n$-Trp where n is from 1 to 4, Pro-Val-Phe-Val, Val-Phe-Val, or absent; each R1 and R2, independently, is H, $C_{1-12}$ alkyl, $C_{7-10}$ phenylalkyl, or C (=O) $E_1$, where $E_1$ is $C_{1-20}$ alkyl, $C_{3-20}$ alkenyl, $C_{3-20}$ alkynyl, phenyl, 3,4-dihydroxyphenylalkyl, naphthyl, or $C_{7-10}$ phenylalkyl; provided that when either R1 or R2 is C (=O) $E_1$, the other must be H; R3 is OH, $NH_2$, $C_{1-12}$ alkoxy, $C_{7-10}$ phenylalkoxy, $C_{11-20}$ naphthylalkoxy, $C_{1-12}$ alkylamino, $C_{7-10}$ phenylalkylamino, or $C_{11-20}$ naphthyla-lkylamino; and n is 1 or 2.

13. A composition of claim 12, wherein said composition further comprises a water-soluble film-former.

14. A composition of claim 13, wherein said water-soluble film-former is selected from the group consisting of gums, proteins, and polymers.

15. A composition of claim 12, wherein said composition further comprises an oil-soluble film-former.

16. A composition of claim 13, wherein said composition further comprises an oil-soluble film-former.

17. A composition of claim 15, wherein said oil-soluble film-former selected from the group consisting of adipic acid/ diethylene glycol/glycerin crosspolymer, acrylates/dimethicone copolymer, trimethyl siloxysilicate, VP/hexadecene copolymer, and TVP/eicocene copolymer.

18. A composition of claim 16, wherein said oil-soluble film-former selected from the group consisting of adipic acid/ diethylene glycol/glycerin crosspolymer, acrylates/dimethicone copolymer, trimethyl siloxysilicate, VP/hexadecene copolymer, and TVP/eicocene copolymer.

19. A composition of claim 12, wherein the external phase of said emulsion comprises a dimethicone crosspolymer.

20. A composition of claim 12, wherein the external phase of said emulsion comprises a dimethicone crosspolymer.

21. A method of reducing the appearance of wrinkles on the skin, said method comprising applying the composition of claim 1 to the skin of a subject in need of such reduction.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 4665054 A
- US 4760051 A
- US 4810693 A
- US 4877770 A
- US 5135913 A
- US 5348943 A
- US 5382431 A
- US 5550183 A